(19) Europäisches Patentamt
European Patent Office
Office européen des brevets

(11) **EP 2 883 987 B1**

(12) **EUROPÄISCHE PATENTSCHRIFT**

(45) Veröffentlichungstag und Bekanntmachung des Hinweises auf die Patenterteilung:
**20.09.2017 Patentblatt 2017/38**

(51) Int Cl.:
*A61F 13/53* *(2006.01)*  *A61F 13/15* *(2006.01)*
*A61L 15/22* *(2006.01)*  *D01F 1/08* *(2006.01)*

(21) Anmeldenummer: **13196502.2**

(22) Anmeldetag: **10.12.2013**

(54) **Verfahren zur Herstellung eines Vliesstoffes und Vliesstoff**

A process for producing a nonwoven fabric and nonwoven fabric

Procédé de fabrication d'un tissu non tissé et un tissu non tissé

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**17.06.2015 Patentblatt 2015/25**

(73) Patentinhaber: **Reifenhäuser GmbH & Co. KG Maschinenfabrik**
**53839 Troisdorf (DE)**

(72) Erfinder: **Cinquemani, Claudio**
**50733 Köln (DE)**

(74) Vertreter: **Rohmann, Michael**
**Andrejewski - Honke**
**Patent- und Rechtsanwälte**
**An der Reichsbank 8**
**45127 Essen (DE)**

(56) Entgegenhaltungen:
**EP-A1- 2 093 313   US-A- 4 485 141**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Herstellung eines Vliesstoffes aus Endlosfilamenten aus Kunststoff, insbesondere aus thermoplastischem Kunststoff als Depoteinrichtung bzw. als Speichereinrichtung für zumindest ein Wirkmedium, insbesondere für zumindest ein flüssiges Wirkmedium. Die Erfindung betrifft weiterhin einen entsprechenden Vliesstoff aus Filamenten aus Kunststoff, insbesondere aus thermoplastischem Kunststoff.

Verfahren der vorstehend genannten Art sind aus der Praxis in unterschiedlichen Ausführungsformen bekannt. Grundsätzlich ist es auch bekannt, die Eigenschaften von Vliesstoffen zu modifizieren. Es sind zum Beispiel Verfahren bekannt, bei denen die Fasern aufgeschäumt werden. Beispielsweise beschreibt die US 4,485,141 die Herstellung eines Vliesstoffes aus Polyolefinfasern, wobei eine Komponente dieser Fasern aufgeschäumt wird. Auf diese Weise entstehen Poren und es resultiert ein Vliesstoff mit vorteilhaften Eigenschaften insbesondere im Hinblick auf den Griff bzw. das Empfinden beim Berühren des Vliesstoffes. Auch die Druckschrift EP 2 093 313 A1 betrifft aufgeschäumte Fasern bzw. einen Vliesstoff aus aufgeschäumten Fasern. Diese aufgeschäumten Fasern besitzen eine reduzierte Dichte. So kann ein Vliesstoff erhalten werden, der sich durch verbesserte Barriere- und Dämmeigenschaften auszeichnet.

Es ist fernerhin bekannt und wünschenswert die Eigenschaften von Vliesstoffen mit Hilfe spezieller Wirkmedien zu modifizieren. Beispielsweise ist es für den Einsatz von Vliesstoffen in Hygieneprodukten bzw. Sanitärprodukten erstrebenswert, diese Vliesstoffe hydrophil zu modifizieren. Es kann auch wünschenswert sein, die Vliesstoffe hydrophob zu verändern. Prinzipiell bekannt ist es, insbesondere auf einen Vliesstoff aus Fasern aus Polypropylen oder dergleichen Kunststoffen wirkstoffmedienhaltige flüssige Medien aufzubringen.

Ein Vliesstoff aus Polypropylenfasern hat zunächst hydrophobe Eigenschaften. Zur Einstellung hydrophiler Eigenschaften eines solchen Vliesstoffes ist es bekannt, auf diesen Vliesstoff sogenannte permanente Avivagen aufzubringen. Mit dem Begriff "permanent" wird zum Ausdruck gebracht, dass die Avivage bzw. das Wirkmedium nicht ohne Weiteres bzw. kurzfristig von den Vliesstoff beaufschlagenden wässrigen Medien oder dergleichen ausgewaschen wird. Bei erfolgreicher hydrophiler Modifizierung eines solchen Vliesstoffes wird eine verhältnismäßig kurze Durchtrittszeit von wässrigen Medien durch diesen Vliesstoff gewährleistet. Eine solche kurze Durchtrittszeit (sogenannte strikethrough time) ist beispielsweise in Vliesstofflagen für Windeln wünschenswert. Hier ist es vorteilhaft, wenn die Durchtrittszeit von Urin durch die Vlieslage relativ gering ist und möglichst auch längerfristig bzw. bei wiederholter Beaufschlagung verhältnismäßig gering ist. Zur hydrophilen Modifizierung solcher Vlieslagen sind permanente Avivagen bekannt, die insbesondere in Form von Emulsionen auf die Vliesstofflage aufgebracht werden. Dabei finden in der Regel an der Oberfläche der Fasern des Vliesstoffes Vernetzungsreaktionen mit Pfropfung von aktiven bzw. hydrophilen Komponenten statt. Für eine zufriedenstellende Aufbringung müssen die Bedingungen für die Vernetzungsreaktionen und insbesondere die Tröpfchengröße der Emulsionen gezielt eingestellt werden. Diese Verfahrensweise ist sehr aufwendig und kostspielig.

Grundsätzlich könnten zur Realisierung von hydrophilen Eigenschaften auch nicht permanente Avivagen mit hydrophilen Komponenten auf die Fasern bzw. auf die Vliesstoffe aufgebracht werden. Derartige Wirkmedien werden aber durch wässrige Medien wie Urin oder dergleichen relativ schnell aus dem Vliesstoff ausgewaschen und diese Wirkmedien erfüllen dann ihre Funktion in der Regel nur über einen nachteilhaft verhältnismäßig kurzen Zeitraum. - Somit sind die bekannten Maßnahmen verbesserungsfähig bzw. verbesserungsbedürftig.

Demgegenüber liegt der Erfindung das technische Problem zugrunde, ein Verfahren der eingangs genannten Art anzugeben, bei dem die vorstehend geschilderten Nachteile effektiv vermieden werden können. Der Erfindung liegt insbesondere das technische Problem zugrunde, ein Verfahren anzugeben, bei dem eine effektive Depotwirkung bzw. Speicherwirkung für Wirkmedien bzw. flüssige Wirkmedien und vor allem auch für nicht permanente Avivagen erreicht wird. Der Erfindung liegt fernerhin das technische Problem zugrunde, einen entsprechenden Vliesstoff anzugeben.

Zur Lösung des technischen Problems lehrt die Erfindung zunächst ein Verfahren zur Herstellung eines Vliesstoffes aus Endlosfilamenten aus Kunststoff, insbesondere aus thermoplastischem Kunststoff als Depoteinrichtung bzw. als Speichereinrichtung für zumindest ein Wirkmedium, insbesondere für zumindest ein flüssiges Wirkmedium, wobei die Endlosfilamente mittels einer Spinnerette ersponnen werden, wobei der Kunststoff der Endlosfilamente aufgeschäumt wird, so dass zumindest an der Oberfläche bzw. im Oberflächenbereich der Endlosfilamente Poren gebildet werden, wobei dem Kunststoff, insbesondere der Kunststoffschmelze zumindest eine Schäumungssubstanz mit der Maßgabe zugesetzt wird, dass zumindest an der Oberfläche der Endlosfilamente Poren gebildet werden, von denen zumindest 15% nach außen hin offene Poren sind und wobei die offenen Poren zumindest teilweise mit dem Wirkmedium, insbesondere mit dem flüssigen Wirkmedium gefüllt werden. - Die hier und nachfolgend angegebenen Prozentangaben für die nach außen hin offenen Poren beziehen sich insbesondere auf den Volumenanteil der nach außen hin offenen Poren in Bezug auf die Gesamtheit der gebildeten Poren. Der Volumen-%-Anteil der nach außen hin offenen Poren wird vorzugsweise anhand der Aufnahmefähigkeit von Filamenten mit nach außen hin offenen Poren für ein flüssiges Medium im Vergleich zur entsprechenden Aufnahmefähigkeit von nicht geschäumten gleichartigen Filamenten ermittelt. Die Ermittlung kann beispielsweise mit Hilfe des weiter unten noch erläuterten Oil-Pick-Ups erfolgen. - Filamente meint im Rahmen der Erfindung insbesondere Endlosfilamente bzw. Endlosfasern. Diese Endlosfilamente haben sich im Rahmen der Erfindung bzw. zur Lösung des erfindungsgemäßen technischen Problems besonders bewährt. Endlosfilamente unterscheiden sich bekanntlich von Kurzfasern bzw. von sogenannten Stapelfasern. Grundsätzlich könnten im Rahmen des erfindungsgemäßen Verfahrens - jedoch nicht bevorzugt - auch Vliesstoffe aus Stapelfasern eingesetzt werden.

Der Erfindung liegt die Erkenntnis zugrunde, dass sich die erfindungsgemäß erzeugten Vliesstoffe bzw. die erfindungsgemäß realisierte Porenstruktur dieser Vliesstoffe durch eine besonders optimale Speicherkapazität auszeichnen. Wirkmedien bzw. flüssige Wirkmedien können in den Kavitäten der Filamente effektiv und funktionssicher sowie auch langfristig abgelagert werden. Das gilt unter anderem vor allem auch für nicht permanente Avivagen mit hydrophilen Komponenten. Es kann in vorteilhafter Weise eine längerfristige Depotwirkung solcher Wirkmedien erzielt werden.

Nach besonders bevorzugter Ausführungsform des erfindungsgemäßen Verfahrens werden Filamente aus thermoplastischem Kunststoff erzeugt, wobei der thermoplastische Kunststoff empfohlenermaßen aufgeschmolzen wird und die Filamente mit der Spinnerette aus dem schmelzflüssigen Kunststoff ersponnen werden. - Grundsätzlich liegt es aber auch im Rahmen der Erfindung, dass die Filamente durch Lösungsspinnen erzeugt werden. Dabei wird bekanntlich der Kunststoff in einem Lösungsmittel gelöst und die Kunststofflösung wird dann in Form von Filamenten aus der Spinnerette ersponnen.

Eine besonders bevorzugte Ausführungsform des erfindungsgemäßen Verfahrens ist dadurch gekennzeichnet, dass das Wirkmedium bzw. das flüssige Wirkmedium zumindest ein Hydrophilierungsmittel ist oder zumindest ein Hydrophilierungsmittel enthält. Das bevorzugt einzusetzende flüssige Wirkmedium kann dabei eine reine Flüssigkeit oder eine flüssige Lösung oder flüssige Mischung sein. Es liegt im Rahmen der Erfindung, dass eine nicht permanente Avivage als Hydrophilierungsmittel bzw. als flüssiges Wirkmedium auf die Filamente des Vliesstoffes bzw. auf den Vliesstoff aufgebracht wird. Das Hydrophilierungsmittel bzw. die nicht permanente Avivage dient vor allem dem Zweck, den Durchtritt eines wässrigen Mediums - beispielsweise den Durchtritt von Urin oder dergleichen - durch den Vliesstoff zu beschleunigen. Dadurch kann die strikethrough-Zeit für den Durchtritt eines fluiden Mediums bzw. für den Durchtritt eines fluiden wässrigen Mediums durch den Vliesstoff in für viele Anwendungen vorteilhafter Weise verkürzt werden. Das gilt insbesondere für Anwendungen des Vliesstoffes in Hygieneprodukten oder Sanitärprodukten wie beispielsweise Windeln, Binden oder dergleichen. Der Erfindung liegt in diesem Zusammenhang die Erkenntnis zugrunde, dass die erfindungsgemäß erzeugten Poren in den Filamenten des Vliesstoffes - insbesondere die Poren bzw. offenen Poren an der Oberfläche der Filamente - das flüssige Wirkmedium bzw. die Avivage funktionssicher fixieren, so dass eine vorteilhafte kurze strikethrough-Zeit erzielt wird. Das Hydrophilierungsmittel bzw. die Avivage wird langfristig effektiv in den Poren der Filamente gespeichert.

Eine besonders empfohlene Ausführungsform des erfindungsgemäßen Verfahrens zeichnet sich dadurch aus, dass das Wirkmedium bzw. das flüssige Wirkmedium zumindest eine Carboxyl-Verbindung bzw. zumindest eine organische Carboxyl-Verbindung enthält. Das flüssige Wirkmedium - bevorzugt die nicht permanente Avivage - enthält empfohlenermaßen zumindest einen Ester einer Carbonsäure bzw. einen Fettsäureester. Fettsäureester haben sich für nicht permanente Avivagen im Rahmen des erfindungsgemäßen Verfahrens besonders bewährt.

Es liegt im Rahmen der Erfindung, dass die Filamente für den Vliesstoff aus einem thermoplastischen Kunststoff bestehen und aus einer Spinnerette ersponnen werden, wobei insbesondere der thermoplastische Kunststoff aufgeschmolzen wird und die Filamente mittels der Spinnerette aus dem schmelzflüssigen Kunststoff ersponnen werden. Eine besonders empfohlene Ausführungsform des erfindungsgemäßen Verfahrens ist dadurch gekennzeichnet, dass die Filamente bzw. der Vliesstoff aus den Filamenten nach dem Spun-Bond-Verfahren hergestellt werden/wird. Dabei werden vorzugsweise die mit der Spinnerette ersponnenen Filamente durch zumindest eine Kühlkammer geführt und dort mit einem Kühlmedium, bevorzugt mit Kühlluft beaufschlagt. Zweckmäßigerweise werden die Filamente anschließend in eine Verstreckeinheit geführt. Es liegt im Rahmen der Erfindung, dass die gekühlten und verstreckten Filamente auf einer Ablage, insbesondere auf einem Ablagesiebband oder dergleichen zum Vliesstoff bzw. zum Spinnvlies abgelegt werden. Es hat sich im Rahmen des erfindungsgemäßen Verfahrens besonders bewährt, dass die Filamente während dieses Spun-Bond-Verfahrens aerodynamisch verstreckt werden. Zweckmäßigerweise werden die Filamente dabei mit einer Verstreckgeschwindigkeit von 500 m/min bis 4.000 m/min verstreckt. Nach besonders bevorzugter Ausführungsform der Erfindung wird das Spun-Bond-Verfahren in einem sogenannten geschlossenen System durchgeführt. Das bedeutet, dass zweckmäßigerweise im Bereich zwischen Kühlkammer und Verstreckeinheit bzw. dem Ende der Verstreckeinheit mit Ausnahme der Kühlluft keine Zufuhr eines fluiden Mediums bzw. keine Zufuhr von Luft stattfindet. Empfohlenermaßen wird im Rahmen des erfindungsgemäßen Verfahrens das Spinnvlies nach dem sogenannten Reicofil-Verfahren und insbesondere nach dem Reicofil IV-Verfahren als Spun-Bond-Verfahren hergestellt. Empfohlenermaßen ist zwischen der Verstreckeinheit und der Ablageeinrichtung bzw. dem Ablagesiebband zumindest ein Diffusor vorhanden, durch den die verstreckten bzw. aerodynamisch verstreckten Filamente geführt werden, bevor sie auf der Ablageeinrichtung bzw. auf dem Ablagesiebband abgelegt werden. Der Diffusor weist vorzugsweise zur Ablageeinrichtung bzw. zum Ablagesiebband hin zumindest einen divergierenden Abschnitt auf. In diesem divergierenden Abschnitt divergieren die Seitenwände des Diffusors zur Ablageeinrichtung bzw. zum Ablagesiebband hin. - Grundsätzlich könnten die Filamente für das erfindungsgemäße Verfahren auch nach einem Meltblown-Verfahren erzeugt werden. Bevorzugt wird jedoch im Rahmen des erfindungsgemäßen Verfahrens die Durchführung eines Spun-Bond-Verfahrens. Die dabei erzeugten Filamente bzw. Vliesstoffe zeichnen sich im Hinblick auf eine optimale Speicherkapazität für das Wirkmedium und insbesondere für eine Avivage bzw. nicht permanente Avivage durch besonders beachtliche Vorteile aus.

Eine besonders empfohlene Ausführungsform des erfindungsgemäßen Verfahrens ist dadurch gekennzeichnet, dass die Filamente für den Vliesstoff als Multikomponentenfilamente, bevorzugt aus Bikomponentenfilamente hergestellt werden. Dabei werden vorzugsweise die Poren bzw. die nach außen hin offenen Poren in einer an der Oberfläche bzw. Außenoberfläche der Filamente angeordneten Komponente erzeugt. Der Erfindung liegt insoweit die Erkenntnis zugrunde, dass auf diese Weise nicht nur eine effektive Speicherfunktion des Vliesstoffes, sondern auch eine ausreichende Festigkeit bzw. Zugfestigkeit der Filamente gewährleistet werden kann. Nach einer ganz empfohlenen Ausführungsform des erfindungsgemäßen Verfahrens werden die Multikomponentenfilamente bzw. die Bikomponentenfilamente in Kern-Mantel-Konfiguration hergestellt. Dabei werden bevorzugt die Poren bzw. die nach außen hin offenen Poren in der Mantelkomponente gebildet bzw. lediglich in der Mantelkomponente gebildet. Grundsätzlich können im Rahmen der Erfindung die Multikomponentenfilamente bzw. Bikomponentenfilamente auch in Side-by-Side-Konfiguration erzeugt werden und in diesem Fall werden die Poren bzw. die nach außen hin offenen Poren vorzugsweise lediglich in einer Komponente an einer Seite der Filamente gebildet.

Erfindungsgemäß wird dem Kunststoff für die Endlosfilamente, insbesondere der Kunststoffschmelze für die Endlosfilamente zumindest eine Schäumungssubstanz zugesetzt, und zwar mit der Maßgabe zugesetzt, dass zumindest an der Oberfläche der Endlosfilamente Poren gebildet werden, von denen zumindest 15 % nach außen hin offene Poren sind, bevorzugt zumindest 20% und besonders bevorzugt zumindest 25% nach außen hin offene Poren sind. - Zweckmäßigerweise wird der thermoplastische Kunststoff für die Filamente in zumindest einem Extruder aufgeschmolzen. Es liegt im Rahmen der Erfindung, dass die zumindest eine Schäumungssubstanz in dem Extruder zugegeben wird bzw. der Schmelze des thermoplastischen Kunststoffes im Extruder zugefügt wird. Wenn nach bevorzugter Ausführungsform im Rahmen des erfindungsgemäßen Verfahrens Mehrkomponentenfilamente und vor allem Bikomponentenfilamente hergestellt werden, wird die Schäumungssubstanz zweckmäßigerweise nur einer Komponente bzw. nur einer Komponente in dem zugeordneten Extruder zugegeben.

Nach einer Ausführungsform des erfindungsgemäßen Verfahrens wird als Schäumungssubstanz ein Fluid, insbesondere ein komprimiertes Gas bzw. ein komprimiertes inertes Gas zugesetzt. Bei diesem Fluid handelt es sich vorzugsweise um komprimiertes Kohlendioxid oder um komprimierten Stickstoff. Es liegt im Rahmen der Erfindung, dass das komprimierte Fluid im Extruder zugegeben wird bzw. der Schmelze aus dem thermoplastischen Kunststoff im Extruder zugefügt wird. Es liegt weiterhin im Rahmen der Erfindung, dass sich das komprimierte Fluid bei dem im Extruder herrschenden Druck in der Kunststoffschmelze löst. Bei Extrusionsbedingungen im Extruder von 180 bis 300 °C und bei einem Druck von 30 bis 200 bar werden beispielsweise 0,03 bis 0,2 g Kohlendioxid pro Gramm Polypropylen gelöst. - Beim Austritt der Kunststoffschmelze aus der Spinnerette fällt der Druck ab, so dass die Schäumungssubstanz bzw. die physikalische Schäumungssubstanz nicht mehr in Lösung bleiben kann und gasförmig wird. Dabei bilden sich Blasen, die insbesondere an die Filamentoberfläche migrieren. Beim Abkühlen der Schmelze werden die Blasen gleichsam eingefroren und bleiben als Poren bzw. als Porenstruktur in den Filamenten enthalten. - Je nach den Extrusionsbedingungen - insbesondere je nach Druck und/oder Temperatur und/oder Druckabfallgeschwindigkeit und/oder Menge und/oder Art des zugesetzten komprimierten Fluids - kann die Porengröße und Porenanzahl und insbesondere die Menge der nach außen hin offenen Poren sowie die spezifische Oberfläche der Filamente und damit die Speicherkapazität der Filamente gezielt eingestellt werden.

Nach einer anderen bevorzugten Ausführungsform des erfindungsgemäßen Verfahrens wird eine chemisch zersetzbare Schäumungssubstanz zugesetzt bzw. der Schmelze des thermoplastischen Kunststoffes in einem Extruder zugesetzt. Wenn gemäß besonders empfohlener Ausführungsform der Erfindung eine Erzeugung von Multikomponentenfilamenten bzw. insbesondere von Bikomponentenfilamenten erfolgt, wird die chemisch zersetzbare Schäumungssubstanz zweckmäßigerweise nur der Komponente bzw. nur in dem der Komponente zugeordneten Extruder zugesetzt, in der durch Schäumen Poren erzeugt werden sollen. - Bei der chemisch zersetzbaren Schäumungssubstanz handelt es sich insbesondere um zumindest eine Substanz aus der Gruppe "Decarboxylierungssubstanz, Azo-Verbindung, Hydrazin-Verbindung". Es liegt im Rahmen der Erfindung, dass bei Zersetzung der chemisch zersetzbaren Schäumungssubstanz in der Schmelze bzw. in der Kunststoffschmelze ein Gas - insbesondere Kohlendioxid oder Stickstoff - entsteht und somit in der Schmelze Blasen gebildet werden, so dass beim Abkühlen des Kunststoffes bzw. der Filamente Poren bzw. eine Porenstruktur erhalten wird. - Decarboxylierungssubstanz meint im Rahmen der Erfindung eine chemisch zersetzbare Schäumungssubstanz, bei deren chemischer Zersetzung Kohlendioxid entsteht. Nach besonders bevorzugter Ausführungsform des erfindungsgemäßen Verfahrens - aber nicht einschränkend - wird als chemisch zersetzbare Schäumungssubstanz bzw. als Decarboxylierungssubstanz ein Zitronensäurederivat verwendet. Zweckmäßigerweise wird der Schmelze bzw. der Kunststoffschmelze 0,1 Gew.-% bis 10 Gew.-%, vorzugsweise 1 Gew.-% bis 10 Gew.-% eines Masterbatches mit der chemisch zersetzbaren Schäumungssubstanz zugesetzt. Masterbatch meint dabei insbesondere, dass die chemisch zersetzbare Schäumungssubstanz in zumindest einem Kunststoff gelöst ist bzw. mit zumindest einem Kunststoff vermischt ist. Empfohlenermaßen sind 15 Gew.-% bis 50 Gew.-% und bevorzugt 15 Gew.-% bis 45 Gew.-% chemisch zersetzbarer Schäumungssubstanz in dem Kunststoff enthalten. Bei dem Kunststoff handelt es sich nach einer empfohlenen Ausführungsform um ein Polyolefin, insbesondere um ein Polyethylen oder um ein Polypropylen.

Es liegt im Rahmen der Erfindung, dass das Wirkmedium bzw. das flüssige Wirkmedium nach der Ablage der Filamente auf einer Ablageeinrichtung, insbesondere auf einem Ablagesiebband, auf den Vliesstoff aufgebracht wird. Dabei erfolgt das Aufbringen empfohlenermaßen nach der Kalandrierung des Vliesstoffes bzw. nach der Durchführung des Vliesstoffes durch zumindest eine Kalandervorrichtung. Gemäß sehr empfohlener Ausführungsform der Erfindung wird zumindest eine nicht permanente Avivage auf den Vliesstoff bzw. auf den kalandrierten Vliesstoff aufgebracht und bevorzugt eine nicht permanente Avivage als Hydrophilierungsmittel auf den Vliesstoff aufgebracht. Das Aufbringen des Wirkmediums bzw. des flüssigen Wirkmediums kann inline auf den auf der Ablage aufgenommenen Vliesstoff erfolgen oder zu einem späteren Zeitpunkt offline. Nach einer Ausführungsvariante kann ein flüssiges Wirkmedium bzw. eine flüssige Avivage bzw. nicht permanente Avivage mit Hilfe von Sprühdüsen auf den Vliesstoff aufgebracht werden oder auch im Rahmen eines Kiss-roll-Auftragsverfahrens oder im Rahmen eines Schaumauftrags. Es liegt im Rahmen der Erfindung, dass der Vliesstoff mit dem aufgebrachten flüssigen Wirkmedium anschließend getrocknet wird.

Nach sehr empfohlener Ausführungsform des erfindungsgemäßen Verfahrens findet eine Schäumung lediglich an der Oberfläche der Filamente statt, so dass Poren bzw. eine Porenstruktur dann lediglich an der Oberfläche bzw. in Oberflächenbereiche der Filamente vorliegen. Hier hat sich die oben bereits erläuterte bevorzugte Ausführungsform bewährt, nach der Multikomponentenfilamente und insbesondere Bikomponentenfilamente erzeugt werden, bei denen die Schäumung bzw. die Poren lediglich in einer äußeren Komponente bzw. in der Mantelkomponente stattfinden/vorliegen. Zweckmäßigerweise beträgt die Dicke der geschäumten Oberflächenschicht bzw. der porenhaltigen Oberflächenschicht der Filamente 5% bis 50%, bevorzugt 15% bis 40% und besonders bevorzugt 25 % bis 35 % des Filamentdurchmessers. Es liegt im Rahmen der Erfindung, dass der unter der porenhaltigen Oberflächenschicht angeordnete innere Bereich der Filamente ungeschäumt ist und dass dieser innere Bereich keine oder so gut wie keine Poren aufweist.

Gegenstand der Erfindung ist auch ein Vliesstoff bzw. ein Spinnvlies aus Endlosfilamenten, insbesondere aus thermoplastischem Kunststoff, wobei zumindest an der Oberfläche bzw. Außenoberfläche der Endlosfilamente geschäumte Bereiche mit Poren vorhanden sind, von denen zumindest 15%, bevorzugt zumindest 20 % und besonders bevorzugt zumindest 25 % nach außen hin offene Poren sind, wobei die Filamentfeinheit der Endlosfilamente 0,25 den bis 15 den, insbesondere 0,25 den bis 5 den beträgt, wobei die spezifische Oberfläche der Endlosfilamente 20 mm$^2$/m bis 400 mm$^2$/m beträgt und wobei die offenen Poren zumindest teilweise mit einem Wirkmedium, insbesondere mit einem flüssigen Wirkmedium gefüllt sind. Es liegt im Rahmen der Erfindung, dass der Vliesstoff bzw. die Endlosfilamente mit der Maßgabe hergestellt werden bzw. dass die zumindest eine Schäumungssubstanz mit der Maßgabe zugesetzt wird, dass zumindest an der Oberfläche bzw. an der Außenoberfläche der Endlosfilamente die vorstehend genannte Menge von Poren bzw. nach außen hin offenen Poren gebildet wird, so dass die spezifische Oberfläche der Endlosfilamente bei einer Filamentfeinheit von 0,25 den bis 15 den, insbesondere bei einer Filamentfeinheit von 0,25 den bis 5 den 20 mm$^2$/m bis 400 mm$^2$/m beträgt. Vorzugsweise beträgt das Volumen bzw. das Gesamtvolumen der Poren in den geschäumten Bereichen der Filamente 0,5 % bis 60 %, insbesondere 40 % bis 60 %. Zweckmäßigerweise ist der Durchmesser der Poren kleiner oder gleich 15% des Filamentdurchmessers. Es empfiehlt sich, dass der Durchmesser der Poren 1 % bis 12 % des Filamentdurchmessers beträgt. Es liegt im Rahmen der Erfindung, dass die Filamente des Vliesstoffes aus zumindest einem Kunststoff aus der Gruppe "Polyethylen, Polypropylen, Polyester" erzeugt werden. Als Polyester wird bevorzugt Polyethylenterephthalat (PET) eingesetzt. Grundsätzlich können im Rahmen der Erfindung aber auch andere thermoplastische Kunststoffe bzw. Mischungen von thermoplastischen Kunststoffen eingesetzt werden. Zweckmäßigerweise werden die Filamente aus zumindest einem Kunststoff aus der Gruppe "Polypropylen (PP), Polyethylen (HDPE, LDPE, LLDPE), Polystyrol (PS), Acrylnitril-Butadien-Styrol (ABS), Polyethylentherephtalat (PET), Polyethylentherephtalat mit Glykol (PETG), Polycarbonat (PC), thermoplastisches Elastomer (TPE), thermoplastisches Polyurethan (TPU), Mischung von Kautschuk und thermoplastischen Kunststoffen (TPR), Polyamid (PA), Polyvinylchlorid (PVC)" eingesetzt.

Der Erfindung liegt die Erkenntnis zugrunde, dass mit dem erfindungsgemäßen Verfahren bzw. mit dem erfindungsgemäß erzeugten Vliesstoff und der insbesondere an der Oberfläche/Außenoberfläche der Filamente vorhandenen Porenstruktur ganz besonders vorteilhafte Effekte erzielt werden können. Die Filamente bzw. die Vliesstoffe können in hervorragender Weise als Depoteinrichtung bzw. Speichereinrichtung für Wirkmedien und insbesondere für flüssige Wirkmedien eingesetzt werden. Die Speicherkapazität ist dabei überraschend hoch. Die Wirkmedien bzw. flüssigen Wirkmedien können in vorteilhafter Weise langfristig in den Filamenten bzw. Vliesstoffen fixiert werden und somit langfristig die gewünschten Eigenschaften der Filamente/Vliesstoffe gewährleisten. Die erfindungsgemäß erzeugten Vliesstoffe haben sich insbesondere im Zusammenhang mit der Aufbringung von nicht permanenten Avivagen bewährt. Die Avivagenbestandteile werden in der Porenstruktur der Vliesstoffe bzw. Filamente funktionssicher festgehalten, so dass sie auch langfristig gegenüber Auswaschungen, Austragungen oder dergleichen beständig sind. Es wird davon ausgegangen, dass die Avivagenbestandteile zwar von der Oberfläche der Filamente zumindest teilweise abgewaschen bzw. ausgewaschen werden, dass jedoch Avivage aus den Poren gleichsam nachgeliefert wird bzw. aus den Poren an die Oberfläche des Filamentes migriert. Insoweit wird durch die Realisierung der erfindungsgemäßen Maßnahmen eine an sich nicht permanente Avivage gleichsam zur permanenten Avivage. Das ist vor allem bei dem Einsatz von hydrophil modifizierten Vlieslagen besonders vorteilhaft. Die insbesondere mit Hilfe von an sich nicht permanenten Avivagen hydrophil modifizierten Vlieslagen zeichnen sich durch eine kurze bzw. geringe Durchtrittszeit insbesondere gegenüber wässrigen Medien, wie Urin oder dergleichen aus. Diese vorteilhafte Eigenschaft bleibt bei Verwirklichung der erfindungsgemäßen Maßnahmen auch längerfristig und bei wiederholter Beaufschlagung mit dem wässrigen Medium erhalten. Von daher eignen sich diese hydrophil modifizierten Vlieslagen insbesondere für den Einsatz in Windeln, Binden oder dergleichen Hygieneprodukten. Erfindungsgemäß wird bei solchen modifizierten Vlieslagen bzw. hydrophil modifizierten Vlieslagen eine vorteilhaft kurze strikethrough-Zeit erreicht.

Die erfindungsgemäßen Filamente bzw. die erfindungsgemäßen Vliesstoffe können auch in sehr vorteilhafter Weise zur langsamen gleichsam gesteuerten Abgabe eines Wirkmediums ("controlled release") eingesetzt werden. In diesem Zusammenhang ist darauf hinzuweisen, dass die erfindungsgemäß erzeugten Filamente bzw. Vliesstoffe auch als effektive Speicher für andere Wirkmedien verwendet werden können, beispielsweise für medizinische oder pharmazeutische Wirkmedien oder für Hautpflegemittel, Reinigungsmittel oder dergleichen. Die erfindungsgemäß hergestellten Vliesstoffe können mit entsprechenden Wirkmedien problemlos bezüglich ihrer Eigenschaften angepasst werden und auf funktionssichere Weise hydrophil oder auch hydrophob ausgerüstet werden. Wenn derartige Vliesstoffe als Filtermedien eingesetzt werden, kann die Filterleistung aufgrund der Poren in den Filamenten überraschend stark erhöht werden. Aufgrund der relativ hohen spezifischen Oberfläche der erfindungsgemäß hergestellten Vliesstoffe können auch Vlieseigenschaften wie Griff oder Rauheit oder dergleichen gezielt modifiziert werden. Die höhere spezifische Oberfläche bedingt bei Spinnvliesen auch eine bessere Bondierbarkeit beim Bonden der Filamente. Auch beim Verfestigen der Vliesstoffe durch Vernadeln, insbesondere durch Wasserstrahlvernadeln wird eine sehr gute Haftung zwischen den Filamenten erzielt. Außerdem sind die erfindungsgemäß erzeugten Vliesstoffe aufgrund der hohen Oberfläche auf einfache Weise bedruckbar. Im Rahmen des erfindungsgemäßen Verfahrens hergestellte Vliesstoffe können auch für Wischtücher und dergleichen eingesetzt werden. Hervorzuheben ist auch, dass die Vliesstoffe mit dem erfindungsgemäßen Verfahren einfach und kostengünstig herstellbar sind.

Nachfolgend wird die Erfindung anhand einer lediglich ein Ausführungsbeispiel darstellenden Zeichnung näher erläutert. Es zeigen in schematischer Darstellung:

Fig. 1     eine Vorrichtung zur Herstellung eines erfindungsgemäßen Vlies-stoffes in perspektivischer Darstellung,

Fig. 2     eine perspektivische Ansicht eines erfindungsgemäß erzeugten Filamentes,

Fig. 3     einen Schnitt durch den Gegenstand nach Fig. 2 und

Fig. 4     ein erstes Diagramm zum Ausführungsbeispiel und

Fig. 5     ein zweites Diagramm zum Ausführungsbeispiel.

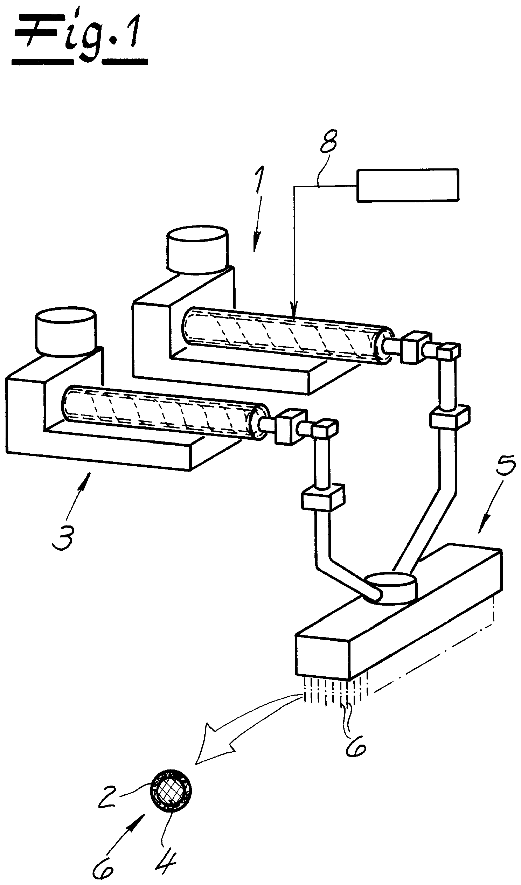

Die Fig. 1 zeigt eine Vorrichtung zur Herstellung eines Spinnvlieses aus Filamenten aus thermoplastischem Kunststoff nach dem Spun-Bond-Verfahren. Die Filamente werden als Bikomponentenfilamente 6 mit Kern-Mantel-Konfiguration erzeugt. Die Vorrichtung weist einen ersten Extruder 1 zum Aufschmelzen des thermoplastischen Kunststoffes für die Mantelkomponente 2 auf sowie einen zweiten Extruder 3 zum Aufschmelzen des thermoplastischen Kunststoffes der Kernkomponente 4. Die beiden Kunststoffschmelzen werden aus den Extrudern 1, 3 herausgeführt und einer Spinnerette 5 zugeführt, aus der die Bikomponentenfilamente 6 in Kern-Mantel-Konfiguration ersponnen werden.

Vorzugsweise und im Ausführungsbeispiel wird die Mantelkomponente 2 aufgeschäumt, so dass Poren 7, insbesondere nach außen hin offene Poren 7a in der Mantelkomponente 2 und an der Oberfläche der Bikomponentenfilamente 6 gebildet werden. Dafür wird zweckmäßigerweise und im Ausführungsbeispiel der Kunststoffschmelze für die Mantelkomponente 2 im ersten Extruder 1 komprimiertes flüssiges Kohlendioxid zugeführt, und zwar über eine Zuführungsleitung 8. Das komprimierte Fluid löst sich bei dem im Extruder 1 herrschenden Druck in der Kunststoffschmelze. Beim Austritt der Kunststoffschmelze aus der Spinnerette 5 fällt der Druck ab und das komprimierte Kohlendioxid kann nicht in Lösung bleiben und wird gasförmig. Dabei werden Blasen erzeugt, die unter anderem an die Oberfläche der Bikomponentenfilamente 6 migrieren. Beim Abkühlen der Kunststoffschmelze werden die Blasen eingefroren und bleiben in Form von Poren 7, 7a in den Filamenten erhalten.

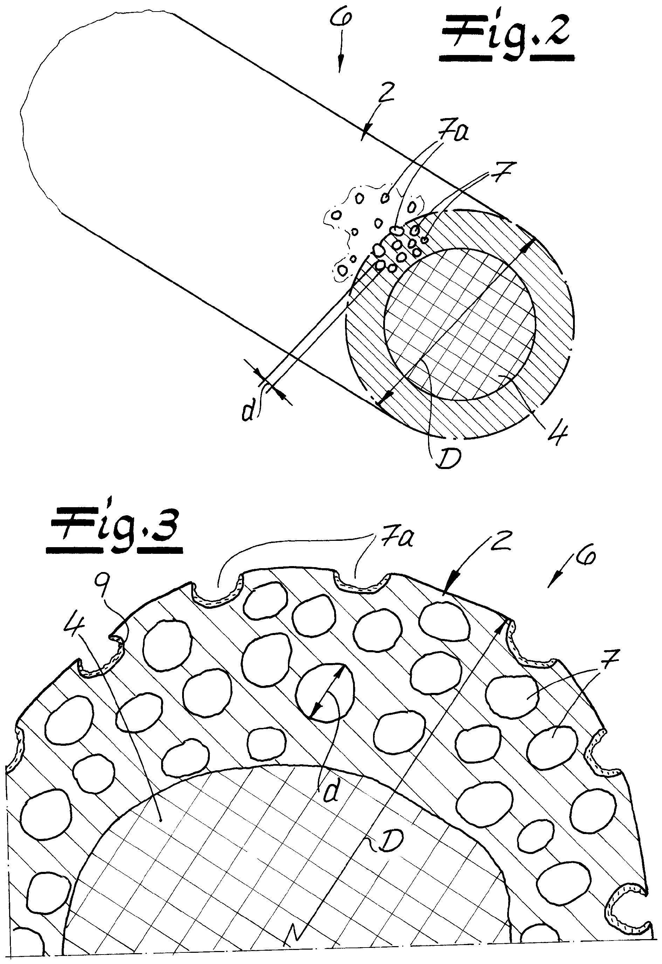

Die Fig. 2 und 3 zeigen ein erfindungsgemäß erzeugtes Bikomponentenfilament 6 mit Poren 7 bzw. offenen Poren 7a in der Mantelkomponente 2. Es ist erkennbar, dass eine Mehrzahl von nach außen hin offenen Poren 7a an der Außenoberfläche der Mantelkomponente 2 angeordnet sind. Dadurch ergibt sich eine vorteilhaft hohe spezifische Oberfläche der Bikomponentenfilamente 6. Vorzugsweise sind von den an der Oberfläche bzw. Außenoberfläche der Bikomponentenfilamente 6 gebildeten Poren 7 zumindest 20 %, bevorzugt zumindest 30 % nach außen offene Poren 7a. - Der Porendurchmesser d beträgt empfohlenermaßen 1 % bis 12 % des Durchmessers D der Bikomponentenfilamente 6. Im Ausführungsbeispiel mag der Porendurchmesser d zwischen 2,2 und 4 μm betragen. In der Fig. 3 sind im Übrigen separate bzw. voneinander getrennte Poren 7 dargestellt. Grundsätzlich können die Poren 7, 7a zumindest teilweise auch untereinander verbunden sein.

Besondere Bedeutung kommt im Rahmen der Erfindung der an der Filamentoberfläche vorhandenen nach außen hin offenen Poren 7a zu. Insbesondere in diesen nach außen hin offenen Poren kann ein Wirkmedium bzw. ein fluides Wirkmedium funktionssicher aufgenommen und fixiert werden. Im Ausführungsbeispiel (Fig. 3) ist in diesen nach außen hin offenen Poren 7a der Bikomponentenfilamente 6 eine nicht permanente Avivage 9 als Hydrophilierungsmittel aufgenommen. Dadurch werden an sich hydrophobe Filamente bzw. Vliesstoffe hydrophil modifiziert. Die nicht permanente Avivage 9 wird insbesondere in den nach außen hin offenen Poren 7a des Vliesstoffes funktionssicher fixiert, so dass sie auch langfristig im Wesentlichen gegenüber Auswaschungen, Austragungen und dergleichen resistent ist. Es wird davon ausgegangen, dass die Avivage zwar von der porenfreien Filamentoberfläche zumindest teilweise abgewaschen bzw. ausgewaschen wird, dass jedoch Avivage aus den offenen Poren 7a nachgeliefert wird bzw. gleichsam an die Filamentoberfläche migriert. Dadurch ergibt sich für eine mit der nicht permanenten Avivage 9 ausgerüsteten Vliesstofflage eine vorteilhaft geringe Durchtrittszeit (strikethrough-Zeit) insbesondere für wässrige Medien wie Urin oder dergleichen. Von daher eignet sich eine solche erfindungsgemäß hydrophil modifizierte Vlieslage vor allem für den Einsatz in Windeln und dergleichen Hygieneprodukten.

Ausführungsbeispiel:

Mit einem Spun-Bond-Verfahren, nämlich mit dem Reicofil-IV-Verfahren wurde ein Referenzvlies (1075) mit ungeschäumten Filamenten sowie zwei erfindungsgemäße Spinnvliese (1090 und 1089) mit geschäumten Filamenten hergestellt. Die Filamente wurden aus Polypropylen und zwar aus den handelsüblichen Polypropylen-Rohstoffen 561R erzeugt. In der nachfolgenden Tabelle 1 sind die Einstellungen für das Spun-Bond-Verfahren sowie Messergebnisse für die drei Spinnvliese angegeben.

Die Filamente für die erfindungsgemäßen Spinnvliese wurden mit einer Vorrichtung entsprechend Fig. 1 unter Einsatz der beiden Extruder 1, 3 als Bikomponentenfilamente 6 mit Kern-Mantel-Konfiguration hergestellt. Beide Komponenten bestanden aus dem vorgenannten Polypropylen. Lediglich dem Kunststoff bzw. der Kunststoffschmelze für die Mantelkomponente 2 wurde im ersten Extruder 1 eine chemisch zersetzbare Schäumungssubstanz, nämlich Hydrocerol 40 zugesetzt. Dabei handelt es sich um ein Masterbatch mit einer chemisch zersetzbaren Schäumungssubstanz. Bei diesem Masterbatch sind 40 Gew.-% eines Zitronensäurederivates als chemisch zersetzbare Schäumungssubstanz in Polyethylen (LDPE) gelöst. In der Spalte 2 der Tabelle 1 ist die Konzentration Hydrocerol 40 in der Mantelkomponente 2 in Gew.-% angegeben. Die Schäumung der Mantelkomponente 2 fand unmittelbar beim Austritt der Filamente aus der Spinnerette 5 statt. Das Gewichtsverhältnis Kern/Mantel der geschäumten Filamente betrug im Übrigen 70/30. Auch das Referenz-Spinnvlies wurde mit einer Vorrichtung gemäß Fig. 1 hergestellt. Dabei wurden ebenfalls die beiden Extruder 1, 3 eingesetzt. Hier wurde jedoch keine Schäumungssubstanz zugesetzt.

Aus der Spalte 3 ergibt sich die Extrusionstemperatur für die Filamente aller Spinnvliese. Die Filamente wurden aus einer Spinnerette extrudiert und anschließend durch eine Kühlkammer zum Kühlen der Filamente geführt. Aus der Spalte 4 ergibt sich jeweils die Anblastemperatur für die Kühlkammer. In der Spalte 5 ist die eingestellte Fadengeschwindigkeit bzw. Filamentgeschwindigkeit in m/min angegeben.

Die Gesamtoberfläche (Spalte 6) in m²/g wurde ermittelt, indem der durchschnittliche (n = 10) Durchmesser (Spalten 8 und 9) der auf dem Siebband abgelegten Filamente anhand des Querschnitts gemessen wurde. Mit dem bekannten Durchsatz und der bekannten Filamentanzahl ergibt sich über den optisch bestimmten Titer in [den] die Filamentlänge (Titer in [den] entspricht [g/9000 m]) $\left( T[den] = 0,9d[\mu m]^2 \rho[g/cm^2]\pi\frac{1}{400} \right)$. Die Gesamtoberfläche der ungeschäumten Filamente resultiert aus dem Umfang und der Zylinderlänge. Für ein geschäumtes Filament wurde im Querschnitt die durchschnittliche (n = 10) Anzahl der Poren gezählt und deren durchschnittlicher (n = 10) Durchmesser gemessen. Als Porosität wurden zylinderförmige Poren angenommen. Die Messmethode lässt nur zu, Poren im Querschnitt der Filamente zu bestimmen und somit erscheinen die Poren als kreisförmige Fehlstellen. Wegen der Unterziehung der Filamente ist davon auszugehen, dass aus der am Spinnerettenaustritt kugelförmigen Pore eine zylinderförmige Pore wird, die die maximale Länge von Porendurchmesser x Verzug hat.

Die Porosität (Spalten 10 und 11) wurde errechnet, indem die summierte Querschnittsfläche aller Poren einer Schnittebene in Relation gesetzt wurde zur Querschnittsfläche des Gesamtfilamentes bzw. nur der Mantelfläche.

Das Aufziehverhalten wurde anhand des Oil-pick-ups (OPU) mit Silastol 163 in wässriger Emulsion ermittelt. Dabei handelt es sich um eine nicht permanente Avivage aus hydrophilen Komponenten und Netzmitteln. Der gravimetrisch bestimmte OPU wurde aus dem Verhältnis der Masse der Spinnvliese ohne Avivage und der Masse der getrockneten Spinnvliese mit Avivage berechnet.

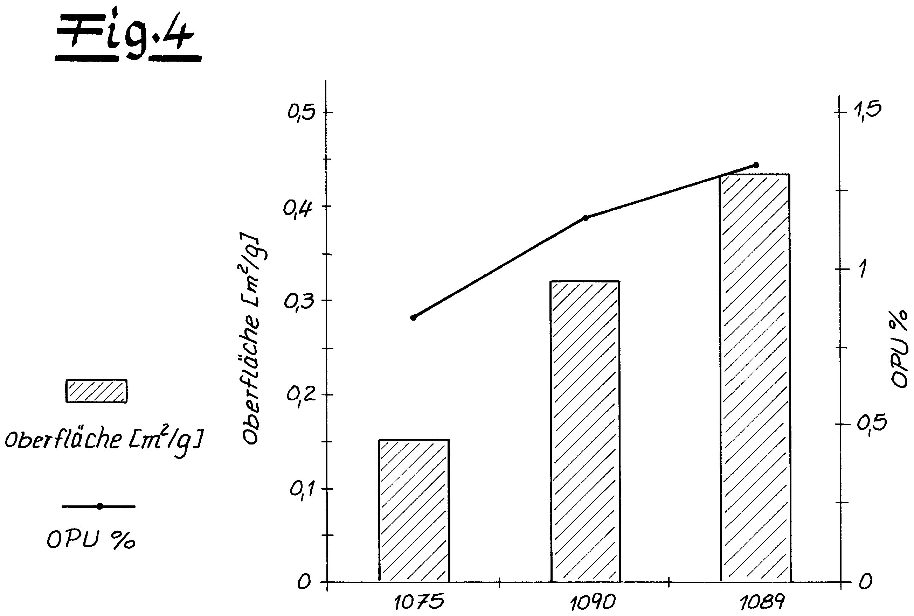

Die Fig. 4 zeigt für die drei Spinnvliese (1075, 1090 und 1089) einerseits die Gesamtoberfläche in $m^2/g$ und andererseits den OPU in Prozent. Aus der Fig. 4 und der Tabelle 1 ist entnehmbar, dass die Gesamtoberfläche des ersten Spinnvlieses (1090) mit geschäumten Filamenten im Vergleich zum Spinnvlies mit ungeschäumten Filamenten (1075) um mehr als 100 % vergrößert ist. Bei dem zweiten Spinnvlies (1089) mit geschäumten Filamenten ist die Gesamtoberfläche gegenüber dem Spinnvlies (1075) mit ungeschäumten Filamenten fast verdreifacht. Die Vergrößerung der Gesamtoberflächen korreliert mit der Erhöhung der Aufnahmefähigkeit für die Avivage. Der OPU wird von 0,84 % bei dem Spinnvlies (1075) mit ungeschäumten Filamenten auf 1,17 % bzw. 1,33 % bei den Spinnvliesen (1089 und 1090) mit geschäumten Filamenten erhöht.

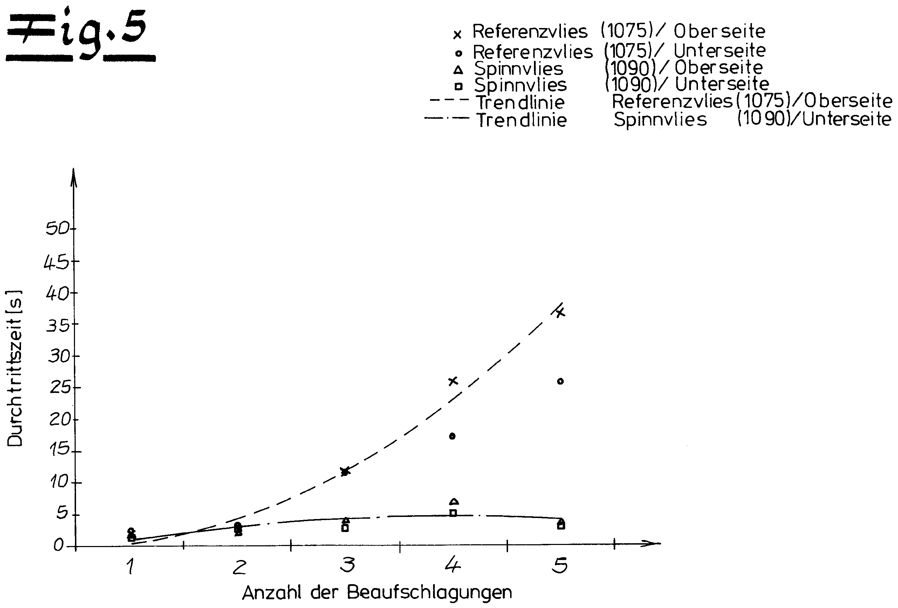

Die Fig. 5 zeigt die Messergebnisse für einen multiple-strikethrough-Test für ein Referenzvlies (1075) einerseits und für ein erfindungsgemäß erzeugtes Spinnvlies (1090) andererseits. Dieser Test ist in ISO 9073-8 (1995) näher spezifiziert. Bei diesem Test wird die Durchtrittszeit (strikethrough time) durch das zu testende Spinnvlies (hier: 1075 und 1090) gemessen. Dabei wird ein definiertes Volumen einer Flüssigkeit (simuliertes Urin: hier 0,9 Gew.-%ige wässrige Kochsalzlösung) auf die Vliesoberfläche gebracht. Unter dem Spinnvlies befindet sich in Kontakt mit dem Spinnvlies ein standardisiertes absorbierendes Zellulose-Kissen, das die durch das Spinnvlies tretende Flüssigkeit aufnimmt. Die Beaufschlagung eines Spinnvlieses wurde jeweils fünfmal mit je 5 ml der wässrigen Lösung durchgeführt, und zwar sowohl von der Oberseite des Spinnvlieses aus als auch von der Unterseite des Spinnvlieses aus. - In dem Diagramm gemäß Fig. 5 ist die Durchtrittszeit (strikethrough time) in Abhängigkeit von der Anzahl der Beaufschlagungen des Spinnvlieses dargestellt. Die gestrichelte Kurve steht für das Referenzvlies (1075) mit ungeschäumten Filamenten. Hier wird das Hydrophilierungsmittel bzw. die nicht permanente Avivage bei mehrmaliger Beaufschlagung des Spinnvlieses schnell verbraucht und dadurch verlängert sich die Durchtrittszeit bzw. strikethrough time durch das Spinnvlies deutlich. Dagegen steht die strichpunktierte Kurve für ein erfindungsgemäßes Vlies (1090) mit geschäumten Filamenten. Es ist erkennbar, dass auch bei mehrmaliger Beaufschlagung eine niedrige Durchtrittszeit gewährleistet bleibt. Hier wird das Hydrophilierungsmittel bzw. die nicht permanente Avivage in den Poren der Filamente wirksam gespeichert und somit können die hydrophilen Eigenschaften des Spinnvlieses längerfristig bzw. langfristig gewährleistet werden. Wie oben bereits dargelegt, ist eine derart erfindungsgemäß modifizierte Vliesstofflage insbesondere vorteilhaft für Windeln und dergleichen. Mit einer erfindungsgemäß hydrophil modifizierten Vliesstofflage kann in Windeln das Urin gleichsam schnell wegtransportiert werden und insbesondere einem Saugkern aus Zellulose oder dergleichen zugeführt werden.

Tab. 1 Prozesseinstellungen und Ergebnisse für ein Referenz-Spinnvlies und für zwei erfindungsgemäße Spinnvliese:

| Prozess | | | | | | Ergebnisse | | | | | | |
|---|---|---|---|---|---|---|---|---|---|---|---|---|
| Spaltennr. | 1 | 2 | 3 | 4 | 5 | 6 | 7 | 8 | 9 | 10 | 11 | 12 |
| Erläuterung | Proben-Nr. | Konz Hydrocerol 40 | Extrus.-Temp. | Anblas-Temp. | Eingestellte Fadengeschwindigkeit | Gesamtoberfläche | Gesamtoberfläche | Filamentdurchmesser | Filamentdurchmesser | Porenfläche/Gesamtquerschnittsfläche | Porenfläche/Mantelquerschnittsfläche | Gravimetrischer OPU |
| | | Gew.-% | °C | °C | m/min | [m$^2$/g] | [mm$^2$/m] | [µm] | [den] | [%] | [%] | [%] |
| ungeschäumte Filamente | 1075 | 0 | 220 | 20 | 2550 | 0,15 | 97,39 | 31 | 6,2 | 0,00 | 0,00 | 0,84 |
| geschäumte Filamente | 1090 | **4,5** | 220 | 20 | 2550 | 0,32 | 203,87 | 31 | 6,2 | 13,97 | 46,56 | 1,17 |
| geschäumte Filamente | 1089 | **4,5** | 220 | 20 | 2300 | 0,43 | 276,41 | 32 | 6,6 | 21,74 | 72,46 | 1,33 |

EP 2 883 987 B1

**EP 2 883 987 B1**

**Patentansprüche**

1. Verfahren zur Herstellung eines Vliesstoffes aus Endlosfilamenten aus Kunststoff, insbesondere aus thermoplastischem Kunststoff als Depoteinrichtung bzw. Speichereinrichtung für zumindest ein Wirkmedium, insbesondere für zumindest ein flüssiges Wirkmedium, wobei die Endlosfilamente mittels einer Spinnerette (5) ersponnen werden, wobei der Kunststoff der Endlosfilamente aufgeschäumt wird, so dass zumindest an der Oberfläche bzw. im Oberflächenbereich der Endlosfilamente Poren (7, 7a) gebildet werden, wobei dem Kunststoff, insbesondere der Kunststoffschmelze zumindest eine Schäumungssubstanz mit der Maßgabe zugesetzt wird, dass zumindest an der Oberfläche der Endlosfilamente Poren (7a) gebildet werden, von denen zumindest 15 % nach außen hin offene Poren (7a) sind und wobei die offenen Poren (7a) zumindest teilweise mit dem Wirkmedium, insbesondere mit dem flüssigen Wirkmedium gefüllt werden.

2. Verfahren nach Anspruch 1, wobei das Wirkmedium bzw. das flüssige Wirkmedium zumindest ein Hydrophilierungsmittel ist oder zumindest ein Hydrophilierungsmittel enthält.

3. Verfahren nach einem der Ansprüche 1 oder 2, wobei das Wirkmedium bzw. das flüssige Wirkmedium zumindest eine Carboxyl-Verbindung enthält.

4. Verfahren nach einem der Ansprüche 1 bis 3, wobei die Filamente bzw. der Vliesstoff aus den Filamenten nach dem Spun-Bond-Verfahren hergestellt werden/wird und wobei insbesondere die mit der Spinnerette (5) ersponnenen Filamente durch zumindest eine Kühlkammer geführt werden und dort mit Kühlluft beaufschlagt werden und wobei die Filamente vorzugsweise anschließend in eine Verstreckeinheit eingeführt werden.

5. Verfahren nach Anspruch 4, wobei die Filamente mit einer Verstreckgeschwindigkeit von 500 m/min bis 4.000 m/min verstreckt werden.

6. Verfahren nach einem der Ansprüche 1 bis 5, insbesondere nach einem der Ansprüche 4 oder 5, wobei die Filamente als Multikomponentenfilamente, insbesondere als Bikomponentenfilamente (6) hergestellt werden und wobei vorzugsweise die Poren (7, 7a) bzw. die nach außen hin offenen Poren (7a) in einer an der Oberfläche bzw. Außenoberfläche der Filamente angeordneten Komponente erzeugt werden.

7. Verfahren nach Anspruch 6, wobei die Multikomponentenfilamente bzw. die Bikomponentenfilamente (6) in Kern-Mantel-Konfiguration erzeugt werden und wobei die Poren (7, 7a) bzw. die nach außen hin offenen Poren (7a) in der Mantelkomponente bzw. lediglich in der Mantelkomponente gebildet werden.

8. Verfahren nach einem der Ansprüche 1 bis 7, wobei die Schäumungssubstanz mit der Maßgabe zugesetzt wird, dass zumindest an der Oberfläche der Endlosfilamente Poren (7, 7a) gebildet werden, von denen zumindest 20 % und besonders bevorzugt zumindest 25 % nach außen hin offene Poren (7a) sind.

9. Verfahren nach Anspruch 8, wobei die Schäumungssubstanz in einem Extruder (1, 3) zugegeben wird.

10. Verfahren nach einem der Ansprüche 1 bis 9, wobei das Wirkmedium bzw. das flüssige Wirkmedium nach der Ablage der Filamente zum Vliesstoff - insbesondere auf einer Ablageeinrichtung, wie einem Ablagesiebband oder dergleichen - auf den Vliesstoff aufgebracht wird und vorzugsweise nach dem Kalandrieren des Vliesstoffes auf den Vliesstoff aufgebracht wird.

11. Vliesstoff aus Endlosfilamenten aus thermoplastischem Kunststoff, hergestellt gemäß einem Verfahren nach einem der Ansprüche 1 bis 10, wobei zumindest an der Oberfläche bzw. Außenoberfläche der Endlosfilamente geschäumte Bereiche mit Poren (7, 7a) vorhanden sind, von denen zumindest 15%, bevorzugt zumindest 20 % und besonders bevorzugt zumindest 25% nach außen hin offene Poren (7a) sind, wobei die Filamentfeinheit der Endlosfilamente 0,25 den bis 15 den, insbesondere 0,25 den bis 5 den beträgt, wobei die spezifische Oberfläche der Endlosfilamente 20 mm²/m bis 400 mm²/m beträgt und wobei die offenen Poren (7a) zumindest teilweise mit einem Wirkmedium, insbesondere mit einem flüssigen Wirkmedium gefüllt sind.

12. Vliesstoff nach Anspruch 11, wobei das Volumen der Poren (7, 7a) in den geschäumten Bereichen 0,5 % bis 60 %, insbesondere 1 % bis 60 % beträgt.

13. Vliesstoff nach einem der Ansprüche 11 oder 12, wobei der Durchmesser (d) der Poren (7, 7a) kleiner oder gleich 15% des Filamentdurchmessers (D) ist.

14. Vliesstoff nach Anspruch 13, wobei der Durchmesser (d) der Poren (7, 7a) 1 % bis 12 % des Filamentdurchmessers (D) beträgt.

15. Hygieneartikel, insbesondere Windel, Binde oder dergleichen mit zumindest einer Lage aus einem Vliesstoff nach einem der Ansprüche 11 bis 14.

**Claims**

1. Method for producing a nonwoven fabric of endless filaments made of plastic, in particular made of thermoplastic material as a depositing device or storage device for at least one active medium, in particular for at least one liquid active medium wherein the endless filaments are spun by means of a spinneret (5), wherein the plastic of the endless filaments is foamed so that pores (7, 7a) are formed at least on the surface or in the surface area of the endless filaments, wherein at least one foaming substance is added to the plastic, in particular the plastic melt with the proviso that pores (7a) are formed at least on the surface of the endless filaments, of which at least 15% are outwardly open pores (7a) and wherein the open pores (7a) are filled at least partially with the active medium in particular with the liquid active medium.

2. The method according to claim 1, wherein the active medium or the liquid active medium is at least one hydrophilizing agent or contains at least one hydrophilizing agent.

3. The method according to any one of claims 1 or 2, wherein the active medium or the liquid active medium contains at least one carboxyl compound.

4. The method according to any one of claims 1 to 3, wherein the filaments or the nonwoven fabric composed of the filaments are/is produced by the spun bond method and wherein in particular the filaments spun by the spinneret (5) are passed through at least one cooling chamber and there are subjected to cooling air and wherein the filaments are preferably then introduced into a stretching unit.

5. The method according to claim 4, wherein the filaments are stretched at a stretching speed of 500 m/min to 4000 m/min.

6. The method according to any one of claims 1 to 5, in particular according to any one of claims 4 or 5, wherein the filaments are produced as multicomponent filaments, in particular as bicomponent filaments (6) and wherein preferably the pores (7, 7a) or the outwardly open pores (7a) are produced in a component arranged on the surface or outer surface of the filaments.

7. The method according to claim 6, wherein the multicomponent filaments or the bicomponent filaments (6) are produced in a core-sheath configuration and wherein the pores (7, 7a) or the outwardly open pores (7a) are formed in the sheath component or merely in the sheath component.

8. The method according to any one of claims 1 to 7, wherein the foaming substance is added with the proviso that pores (7, 7a) are formed at least on the surface of the endless filaments, of which at least 20% and particularly preferably at least 25% are outwardly open pores (7a).

9. The method according to claim 8, wherein the foaming substance is added in an extruder (1, 3).

10. The method according to any one of claims 1 to 9, wherein the active medium or the liquid active medium is applied to the nonwoven fabric after deposition of the filaments to form the nonwoven fabric - in particular on a depositing device such as a foraminous deposition belt or the like - and preferably after calendering the nonwoven fabric.

11. Nonwoven fabric comprising endless filaments of thermoplastic material produced according to a method according to any one of claims 1 to 10, wherein at least on the surface or outer surface of the endless filaments, foamed regions with pores (7, 7a) are provided of which at least 15%, preferably at least 20% and particularly preferably at least 25% are outwardly open pores (7a), wherein the filament fineness of the endless filaments is 0.25 den to 15 den, in particular 0.25 den to 5 den, wherein the specific surface area of the endless filaments is 20 $mm_2$/m to 400 $mm^2$/m

and wherein the open pores (7a) are at least partially filled with an active medium, in particular with a liquid active medium.

12. The nonwoven fabric according to claim 11, wherein the volume of the pores (7, 7a) in the foamed regions is 0.5% to 60%, in particular 1% to 60%.

13. The nonwoven fabric according to any one of claims 11 or 12, wherein the diameter (d) of the pores (7, 7a) is less than or equal to 15% of the filament diameter (D).

14. The nonwoven fabric according to claim 13, wherein the diameter (d) of the pores (7, 7a) is 1% to 12% of the filament diameter (D).

15. Hygiene article, in particular nappy, sanitary pad or the like having at least one layer of a nonwoven fabric according to any one of claims 11 to 14.


**Revendications**

1. Procédé destiné à la fabrication d'un non-tissé en filaments continus de matière plastique, notamment de matière thermoplastique en tant que système de dépôt ou en tant que système de stockage d'au moins un milieu actif, notamment pour au moins milieu actif liquide, les filaments continus étant filés au moyen d'une busette (5), la matière plastique des filaments continus étant moussée, de sorte qu'au moins sur la surface ou dans la région superficielle des filaments continus se forment des pores (7, 7a), à la matière plastique, notamment à la matière plastique fondue étant ajoutée une substance de moussage avec le critère qu'au moins à la surface des filaments continus se forment des pores (7a) dont au moins 15 % sont des pores (7a) ouverts sur l'extérieur et les pores (7a) ouverts étant remplis au moins en partie avec le milieu actif, notamment avec le milieu actif liquide.

2. Procédé selon la revendication 1, le milieu actif ou le milieu actif liquide étant au moins un agent d'hydrophilisation ou contenant au moins un agent d'hydrophilisation.

3. Procédé selon l'une quelconque des revendications 1 ou 2, le milieu actif ou le milieu actif liquide contenant au moins un composé carboxylique.

4. Procédé selon l'une quelconque des revendications 1 à 3, le filament ou le non-tissé composé des filaments étant fabriqué(s) selon le procédé de filage dit « spun-bond » et notamment les filaments filés avec la busette (5) étant conduits à travers au moins une chambre de refroidissement et y étant soumis à de l'air de refroidissement et les filaments étant de préférence introduits ensuite dans une unité d'étirage.

5. Procédé selon la revendication 4, les filaments étant étirés à une vitesse d'étirage de 500 m/mn à 4.000 m/mn.

6. Procédé selon l'une quelconque des revendications 1 à 5, notamment selon l'une quelconque des revendications 4 ou 5, les filaments étant fabriqués en tant que filaments multicomposants, notamment en tant que filaments bicomposants (6) et de préférence les pores (7, 7a) ou les pores (7a) ouverts sur l'extérieur étant créés dans un composant placé sur la surface oiu sur la surface extérieure dees filaments.

7. Procédé selon la revendication 6, les filaments multicomposants, ou les filaments bicomposants (6) étant créés en configuration âme-gaine et les pores (7, 7a) ou les pores (7a) ouverts sur l'extérieur étant créés dans la composante gaine ou uniquement dans la composante gaine.

8. Procédé selon l'une quelconque des revendications 1 à 7, la substance de moussage étant ajoutée avec le critère qu'au moins à la surface des filaments continus se forment des pores (7, 7a) dont au moins 20 % et de manière particulièrement préférentielle, au moins 25 % sont des pores (7a) ouverts sur l'extérieur.

9. Procédé selon la revendication 8, la substance de moussage étant ajoutée dans une extrudeuse (1, 3).

10. Procédé selon l'une quelconque des revendications 1 à 9, après la dépose des filaments pour constituer le non-tissé (notamment sur un dispositif de dépose, comme une toile de dépose perforée), le milieu actif ou le milieu actif liquide étant appliqué sur le non-tissé et de préférence étant appliqué sur le non-tissé après le calandrage du non-tissé.

11. Non-tissé composé de filaments continus en matière thermoplastique, fabriqué d'après un procédé selon l'une quelconque des revendications 1 à 10, au moins sur la surface ou sur la surface extérieure des filaments continus étant présentes des régions moussées avec des pores (7, 7a) dont au moins 15 %, de préférence au moins 20 % et de manière particulièrement préférentielle, au moins 25 % sont des pores (7a) dirigés vers l'extérieur, la finesse des filaments continus étant de 0,25 den à 15 den, notamment de 0,25 den à 5 den, la surface spécifique des filaments continus étant de 20 mm$^2$/m à 400 mm$^2$/m et les pores ouverts (7a) étant remplis au moins en partie d'un milieu actif, notamment d'un milieu milieu actif liquide.

12. Non-tissé selon la revendication 11, le volume des pores (7, 7a) dans les régions moussées étant de 0,5 % à 60 %, notamment de 1 % à 60 %.

13. Non-tissé selon l'une quelconque des revendications 11 ou 12, le diamètre (d) des pores (7, 7a) étant inférieur ou égal à 15 % du diamètre des filaments (D).

14. Non-tissé selon la revendication 13, le diamètre (d) des pores (7, 7a) s'élevant à de 1 % à 12 % du diamètre des filaments (D).

15. Article d'hygiène, notamment couche ou similaire avec au moins une nappe en non-tissé selon l'une quelconque des revendications 11 à 14.

# $\mathcal{F}ig.1$

Fig.2

Fig.3

Fig.4

# Fig.5

x Referenzvlies (1075)/ Oberseite
o Referenzvlies (1075)/ Unterseite
△ Spinnvlies (1090)/ Oberseite
□ Spinnvlies (1090)/ Unterseite
--- Trendlinie Referenzvlies (1075)/Oberseite
—·— Trendlinie Spinnvlies (1090)/Unterseite

Durchtrittszeit [s]

50
45
40
35
30
25
20
15
10
5
0

1    2    3    4    5

Anzahl der Beaufschlagungen

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4485141 A
- EP 2093313 A1